# FASCICULE DE BREVET EUROPEEN

(11) **EP 2 903 964 B1**
(45) Date de publication et mention de la délivrance du brevet: **08.08.2018**
(21) Numéro de dépôt: 13782783.8
(22) Date de dépôt: 02.10.2013
(51) Int. Cl.: C07C 235/06, C08K 5/20, C07C 235/10

(54) **AMIDE D'ACIDE GRAS A BASE D'ACIDE 14-HYDROXY-EICOSANOIQUE, COMME ORGANOGELATEUR.**
14-HYDROXY-ARACHINSÄURE-BASIERTES FETTSÄUREAMID ALS ORGANISCH GELIERENDES MITTEL
14-HYDROXYEICOSANOIC ACID-BASED FATTY ACID AMIDE, AS AN ORGANOGELLING AGENT

(30) Priorité: 05.10.2012 FR 1259491
(43) Date de publication de la demande: 12.08.2015
(73) Titulaire: Arkema France, 92700 Colombes (FR)
(72) Inventeur: BERNARD, Michael Y., F-95880 Enghien-les-bains (FR); DUQUENNE, Christophe, F-75010 Paris (FR); DUBOIS, Jean-Luc, F-69390 Millery (FR)
(74) Mandataire: Killis, Andréas
(86) Numéro de dépôt international: PCT/FR2013/052346
(87) Numéro de publication internationale: WO 2014/053774

(56) Documents cités:
- EP-A1- 2 098 502
- WO-A1-2010/100939
- US-A- 4 128 436
- US-A1- 2011 251 294
- US-A1- 2012 129 735
- HAGEMANN: "Thermal behavior of prospective hydroxy acid grease thickeners", JOURNAL OF THE AMERICAN OIL CHEMISTS' SOCIETY, SPRINGER, DE, vol. 68, no. 3, 1 janvier 1991 (1991-01-01), pages 139-143, XP001525982, ISSN: 0003-021X

## Description

L'invention concerne un diamide d'acide gras à base d'acide 14-hydroxy-eicosanoïque et son utilisation comme organogélateur ou agent, appelé aussi additif de rhéologie, en particulier dans des compositions de revêtements, de moulage, de mastics ou d'agents d'étanchéité ou de cosmétique.

Les amides d'acides gras et, en particulier, les diamides à base d'acide 12-hydroxy-stéarique sont déjà connus comme agents organogélateurs, c'est-à-dire des petites molécules organiques capables de gélifier toutes sortes de solvants organiques même à des concentrations massiques relativement faibles (moins de 1% en masse) ou comme additifs de rhéologie, c'est-à-dire permettant de modifier la rhéologie d'une formulation d'application. Ils permettent d'obtenir, par exemple, un effet thixotropique ou pseudoplastique.

US 2011/251294 décrit des dérivés monoamides, monoamines ou monoacides en C₁₈, en particulier hydroxylés en position 12 en tant que gélateurs de faible poids moléculaire pour diverses applications dans divers solvants. Ce document ne décrit, ni ne suggère un amide en acide 14-hydroxy eicosanoïque, noté aussi par la suite par 14-HEA ou 14 HEA, ni aucune performance liée à un tel hydroxyacide.

EP 2 098 502 décrit des mono-, di- ou tetra-amides à base de monoamines ou monoacides en C₃ à C₂₀₀ et plus particulièrement des diamides à base de diamine ou de diacide cyclique en 1,2-cyclohexane et en fonction, de monoacide ou monoamine en C₃ à C₂₀₀, convenables comme des véhicules pour encres solides thermofusibles, à changement de phase lors de l'impression à une température supérieure à la température de fusion de l'encre solide. Le rôle de ces amides en tant que véhicules pour encres solides est de réduire le nombre de composants et donc le coût de l'encre et de rendre la composition d'encre solide uniforme. Aucune performance d'organogélateur en milieu solvant n'est décrite ou suggérée pour ces amides, ni n'est décrit ou suggéré comme tel un amide à base de 14-HEA.

WO 2010/100939 décrit un mélange de diamides de structure cycloaliphatique à base de diamine ou de diacide 1,2-cyclohexane et de monoamines ou de monoacides en C₁₅ à C₂₁, en tant qu'agents gélifiants pour huile. Ce document ne décrit pas, ni ne suggère en tant qu'organogélateur un amide à base de 14-HEA.

US 4,128,436 décrit un diamide de structure oligomérique (oligoamide) à base d'acide hydroxy stéarique, d'une diamine primaire aliphatique saturée et d'un diacide en C₂-C₁₀ ou d'un diacide dimère hydrogéné d'acide gras, comme agent de contrôle rhéologique. Aucun amide à base de 14-HEA n'est décrit ou suggéré en particulier comme organogélateur.

US 2012/129735 décrit des diamides à base d'une diamine primaire et d'un monoacide carboxylique en C₁₂-C₂₂, comme additif de fluide de forage pour puits de pétrole, pour contrôler la rhéologie, en particulier réduire l'augmentation de viscosité en tête du puits de forage avec zone plus froide lors du pompage du pétrole extrait. Aucune mention ou suggestion d'organogélateur à base d'amide dérivé de 14-HEA n'est faite.

L'article intitulé « Thermal Behavior of Prospective Hydroxy Acid Grease Thickeners » dans JAOCS, 68, 3, 139-143 (1991) décrit des sels métalliques, en particulier de lithium, d'hydroxyacides gras et leur comportement thermique et suggère, mais sans exemplification et preuve expérimentale de faisabilité, leur utilisation comme potentiels gélifiants pour graisses à fonction lubrifiante. En fait, cette suggestion se limite aux seuls sels métalliques examinés sur leur comportement thermique qui se distinguent du comportement des acides correspondants non salifiés. Aucune mention ou suggestion particulière d'amide à base de 14-HEA n'est faite en tant qu'organogélateur.

L'acide 12-hydroxy-stéarique, noté aussi 12HSA ou 12-HSA pour la suite, est déjà couramment utilisé comme matière première pour la préparation d'amides gras. Cependant, cet hydroxy acide carboxylique est issu d'une filière dont la source unique est l'huile de ricin. En raison du développement rapide de certaines applications utilisant largement l'huile de ricin directement ou sous forme de ses dérivés, sa consommation a considérablement augmenté, générant des problèmes de disponibilité et de tension sur les prix de ces matières premières issues de la filière huile de ricin, comme le 12-HSA. Il y a donc un besoin croissant de trouver une solution alternative au 12-HSA en cherchant de nouvelles matières premières issues d'une filière indépendante de l'huile de ricin, laquelle filière soit à la fois abondante et également d'origine renouvelable (ou biosourcée), pouvant remplacer partiellement ou complètement, de préférence complètement, le 12-HSA avec des performances d'organogélateur ou rhéologiques satisfaisantes. Un organogélateur est un additif, lequel par définition permet l'obtention d'un gel réversible en milieu solvant organique. Ceci est permis par l'organisation moléculaire spécifique du système aboutissant à une structure fine de réseau, par interactions spécifiques entre les molécules de l'organogélateur d'une part et par les interactions dudit solvant avec ladite molécule de l'organogélateur d'autre part. La réversibilité est obtenue en jouant sur le taux de cisaillement et la température, de sorte que l'augmentation du taux de cisaillement et/ou de la température permet la destruction réversible (par variation inverse) de cette structure fine de réseau.

La présente invention vise de nouveaux diamides gras avec un taux d'acide 12-hydroxy-stéarique (12-HSA), significativement réduit et de préférence nul. Ceci est réalisé en remplaçant le 12-HSA partiellement et de préférence totalement par un hydroxyacide carboxylique gras saturé spécifique : l'acide 14-hydroxy-eicosanoïque (noté aussi 14-HEA ou 14HEA pour la suite), issu de l'huile lesquérolique produite par extraction à partir des graines du Lesquerella et donc de la culture du Lesquerella. Ceci est réalisé tout en gardant des performances d'agent organogélateur tout à fait satisfaisantes, en particulier en termes de pouvoir thixotrope et pouvant, dans certains cas, être aussi bonnes que celles des organogélateurs de l'état de la technique, comme des diamides exclusivement basés sur le 12-HSA et même meilleurs par rapport à certains organogélateurs issus d'hydroxyacides isomères au 12-HSA. L'acide 14-hydroxy-eicosanoïque est issu de l'huile lesquérolique extraite des graines de Lesquerella. Il peut être préparé par transestérification (au méthanol) de ladite huile suivie de l'hydrogénation du produit de transestérification et finalement d'hydrolyse dudit ester hydrogéné pour obtenir l'acide 14-hydroxy-eicosanoïque. Le taux d'ester méthylique de 14-HEA est enrichi par extraction sélective liquide-liquide du mélange d'esters méthyliques. L'acide lesquérolique, présent sous forme d'ester avant hydrogénation, est l'acide 14-hydroxy-eicosen-11 oïque. Un procédé d'obtention d'acide 14-hydroxy-eicosen-11 oïque dont l'hydrogénation conduit au 14-HEA est décrit dans US 3,057,893 et en particulier dans l'exemple 1. Le remplacement partiel ou total ne doit donc pas affecter les performances rhéologiques des produits amides obtenus, tout en respectant un environnement durable avec des matières premières d'origine renouvelable. L'acide 14-hydroxy-eicosanoïque préparé comme cité ci-haut peut contenir parmi ses impuretés de l'acide 12-hydroxy-stéarique dérivé de la présence d'acide ricinoléïque dans l'huile de Lesquerella utilisée au départ pour sa préparation. On ne tient pas compte ici de cette présence très limitée, en général moins de 3% de 12-HSA liée au 14-HEA (à l'obtention du 14-HEA), quand on considère la présence ou l'absence de 12-HSA, cette dernière présence ou absence signifiant l'absence ou présence de 12-HSA rajouté en plus de celui potentiellement résiduel et lié au 14-HEA.

Dans ce but, le premier objet de la présente invention est un diamide d'acide gras, dans lequel l'acide 12-HSA est remplacé, partiellement et de préférence totalement, c'est-à-dire à 100%, par l'acide 14-hydroxy-eicosanoïque (14-HEA). « Remplacement total » par le 14-HEA signifie ici qu'il n'y a pas d'addition de 12-HSA non lié au 14-HEA, car, comme expliqué ci-haut, le 14-HEA (tel qu'utilisé) peut contenir comme impureté résiduelle potentielle une quantité limitée de 12-HSA. La quantité de 12-HSA correspond donc à la quantité additionnée non liée au 14-HEA.

L'invention concerne également un organogélateur et plus particulièrement un agent de rhéologie comprenant ledit amide et l'utilisation dudit amide comme tel.

Finalement, l'invention couvre également une composition de liant organique comprenant en tant qu'agent de rhéologie au moins un amide selon la présente invention.

Donc, le premier objet de l'invention est un amide d'acide gras qui comprend au moins un produit de réaction d'un mélange réactionnel comprenant :
a) au moins une amine sélectionnée parmi :
   ▪ une amine aliphatique linéaire en C₂ à C₁₂, de préférence en C₂ à C₈ et plus préférentiellement en C₂ à C₆, et/ou
   ▪ une amine cycloaliphatique en C₆ à C₁₈, de préférence en C₆ à C₁₂, et/ou
   ▪ une amine aromatique de préférence en C₆ à C₁₂,
b) l'acide 14-hydroxy-eicosanoïque en l'absence ou en présence d'acide 12-hydroxy-stéarique,
c) en option, au moins un monoacide sélectionné parmi les acides carboxyliques linéaires saturés et non hydroxylés en C₆ à C₁₈, de préférence en C₆ à C₁₅, plus préférentiellement en C₆ à C₁₂
d) en option, au moins une deuxième amine différente de a), qui peut être en particulier une diamine et/ou une monoamine, sélectionnée parmi les amines aliphatiques linéaires en C₂ à C₁₂, de préférence en C₂ à C₈ et plus préférentiellement en C₂ à C₆, et en ce que ledit amide est un diamide et que lesdites amines a) et d) sont des diamines.
Selon un cas particulier, ledit acide 12-hydroxy-stéarique peut être présent dans ledit amide selon l'invention, et dans ce cas, le taux molaire d'acide 14-hydroxy-eicosanoïque peut varier de 10 à 99%, de préférence de 20 à 99%, plus préférentiellement de 30 à 99% par rapport au composant b), c'est-à-dire tous les hydroxyacides b).

Plus particulièrement, dans ledit amide de l'invention, ledit composant b) est un mélange d'acide 14-hydroxy-eicosanoïque et d'acide 12-hydroxy-stéarique (12HSA ou 12-HSA), de préférence avec un rapport molaire d'acide 14-hydroxy-eicosanoïque (14-HEA) sur 12-HSA allant de 20/80 à 99/1, plus préférentiellement de 30/70 à 99/1.

Selon une autre option qui est la plus préférée, dans ledit amide l'acide 12-hydroxy-stéarique est absent dudit composant b) et il est remplacé totalement, c'est-à-dire à 100% par l'acide 14-hydroxy-eicosanoïque.

Selon l'invention ledit amide est un diamide et lesdites amines a) et en option d), comme définies ci-haut, sont des diamines correspondantes. Ledit amide peut être en particulier un mélange d'amides comme produits de réaction des composants tels que définis. Il peut par exemple correspondre à un mélange d'amines a) et d). Donc, on peut avoir dans le cas de diamines a) et des diamines d), un mélange de diamides.

Selon l'option plus particulière dudit diamide, celui-ci porte 2 fonctions amides à base du même hydroxyacide b) quand 12-HSA est absent ou à base de deux hydroxyacides b) différents qui sont 14-HEA et 12-HSA, quand l'acide 12-hydroxy-stéarique est présent dans ledit diamide. Plus particulièrement dans le cas de l'invention où ledit amide est un diamide, il peut porter une fonction amide à base d'un hydroxyacide b) et une autre fonction à base dudit monoacide c), ce qui signifie que le rapport molaire b/c est de 1/1.

D'une manière générale, le rapport molaire amine/acide (carboxy) peut varier de 0,9 à 1,1 et de préférence correspond au rapport stoechiométrique de 1/1.

Selon une autre possibilité, ledit amide de l'invention comprend au moins 2, de préférence au moins 3 produits de réaction différents tels qu'issus de la réaction dans ledit mélange réactionnel d'une amine a) et possiblement d'une amine d) avec un monoacide b) et possiblement avec un monoacide c). Ainsi, ledit diamide selon l'invention, peut être un mélange de deux ou de trois produits différents de réaction dans ledit mélange, d'une diamine a) et possiblement d'une diamine d) avec lesdits monoacides b) et c) comme décrits ci-avant. Selon un cas plus particulier ledit diamide de l'invention peut comprendre un mélange de produits de formules suivantes :
- b1-a1-c1
- b1-a1-b1
et de préférence :
- b1-a1-c1
- b1-a1-b1
- b1-a2-b1
avec a1 : résidu de diamine selon a) portant deux groupements amides -NHC(=O)-, a2 : résidu de diamine selon d) portant deux groupements amides -NHC(=O)-, b1 : résidu d'acide 14-hydroxy-eicosanoïque (14-HEA) sans groupement carboxy et c1 : résidu de monoacide non hydroxylé selon c) sans groupement carboxy.

Comme exemple d'amines aliphatiques linéaires convenables et préférées pour le composant amine a) qui est une diamine on peut citer : l'éthylène diamine, la propylène diamine, la butylène (ou tetraméthylène) diamine, la pentaméthylène diamine, l'hexaméthylène diamine, de préférence l'éthylène diamine et l'hexaméthylène diamine.

Comme exemple de diamines cycloaliphatiques convenables toujours selon le composant amine a) qui est une diamine on peut citer : la diamine-1,3, -1,4 et -1,2 et en particulier -1,3 ou -1,4 cyclohexane, l'isophorone diamine, la bis (aminométhyl)-1,3, -1,4 ou -1,2 cyclohexane (dérivé de l'hydrogénation de respectivement m-, p-, o- xylylène diamine), de préférence la bis (aminométhyl)-1,3 ou-1,4 cyclohexane, décahydronaphtalène diamine, le bis(3-méthyl, bis (amino-4 cyclohexyl) méthane (BMACM) ou bis (amino-4 cyclohexyl) méthane (BACM), 1-{[4-(aminométhyl)cyclohexyl]oxy}propan-2-amine. Les diamines cycloaliphatiques préférées sont parmi: diamine-1,3, -1,4 cyclohexane, bis (aminométhyl)-1,3, -1,4 ou -1,2 cyclohexane, isophorone diamine, bis (amino-4 cyclohexyl) méthane.

Comme exemple convenable et préféré de diamines aromatiques en tant que composant amine a) diamine on peut citer : la m-, p-xylylène diamine, la m-, p- phénylène diamine et la m-, p- toluylène diamine.

Comme exemples de monoacides c), on peut citer : l'acide hexanoïque, heptanoïque, octanoïque, nonanoïque, décanoïque, undécanoïque, dodécanoïque, appelé aussi laurique ou le stéarique. Sont préférés : l'hexanoïque, l'octanoïque, le nonanoïque et le décanoïque.

La deuxième amine d) qui est optionnelle est telle que définie pour les amines a) aliphatiques linéaires ci-haut, sauf que dans ce choix l'amine d) est différente de l'amine a).

Concernant le composant b), il est à base de l'hydroxyacide spécifique qui est l'acide 14-hydroxy-eicosanoïque et peut comprendre en mélange de l'acide 12-hydroxy-stéarique et de préférence b) ne comprend pas l'acide 12-hydroxy stéarique.

L'acide 14-hydroxy-eicosanoïque peut être obtenu à partir des graines des plantes oléagineuses du genre Lesquerella.

L'huile obtenue contient majoritairement des triglycérides d'acide lesquérolique qui est l'acide 14-hydroxy-11-eicosenoïque. Elle contient également, mais en partie minoritaire, de l'huile d'acide auricolique qui est l'acide 14-hydroxy-11,17-eicosadiènoïque. Ces deux acides sous forme d'huile, c'est-à-dire de triglycérides, après traitement spécifique et après hydrogénation, aboutissent au même acide gras saturé hydroxylé : le 14-HEA. Suivant les espèces du genre Lesquerella utilisées comme source d'approvisionnement, les proportions des acides gras sont variables et l'huile peut contenir notamment une part résiduelle d'huile d'acide ricinoleïque qui est l'acide 12-hydroxy-9-octadecénoïque et d'acide densipolique qui est l'acide 12-hydroxy-9,15-octadecadienoïque. Ces deux dernières huiles d'acides gras hydroxylés, au terme du processus de traitement et d'hydrogénation, formeront potentiellement une partie résiduelle de 12-HSA qui ne dépasse pas 3% en poids du produit final récupéré comme l'acide 14-HEA. Il est à noter que ce faible pourcentage de 12-HSA résiduel lié au 14-HEA n'est pas considéré dans l'acide défini selon b) quand on parle de présence ou d'absence de 12-HSA dans ledit composant b). Cela signifie, selon l'invention, que la présence ou absence de 12-HSA dans b) est liée uniquement à l'addition ou ajout de 12-HSA et indépendamment du 14-HEA, c'est-à-dire indépendamment du taux résiduel en 12-HSA dans le 14-HEA utilisé.

La conversion de l'huile lesquérolique en fraction d'acides gras peut se faire par une succession d'étapes dont le nombre et l'enchaînement peuvent varier.

L'huile peut ainsi subir un premier traitement de méthanolyse, c'est-à-dire une transestérification en présence de méthanol. Les esters méthyliques obtenus sont séparés du glycérol et soumis à une extraction liquide-liquide qui permet d'enrichir l'une des phases en esters méthyliques d'acides gras hydroxylés. Cette phase subit une hydrogénation pour aboutir à des esters méthyliques gras saturés et hydroxylés. Lors d'une dernière étape, les esters sont hydrolysés pour libérer les chaînes grasses sous forme d'acides gras carboxyliques libres.

Les étapes de méthanolyse, d'hydrogénation et d'enrichissement en fraction hydroxylée peuvent être indépendantes et peuvent être interverties. Ainsi, l'étape d'hydrogénation pourrait être réalisée directement sur l'huile, sur les esters méthyliques bruts, les esters méthyliques enrichis, ainsi que sur les acides gras libres.

Par ailleurs, dans ces procédés, l'étape d'enrichissement est optionnelle suivant le degré de pureté du 14-HEA que l'on souhaite obtenir.

Un autre objet de l'invention concerne l'utilisation des diamides de l'invention, en tant qu'organogélateur, de préférence en tant qu'agent ou additif de rhéologie et plus particulièrement dans une composition préconcentrée dans un plastifiant ou dans un solvant organique sous forme de pâte préactivée. En particulier, cette utilisation concerne des compositions de revêtements, de colles ou adhésifs, de moulage, de mastics ou d'agents d'étanchéité ou de cosmétique.

Un autre objet concerne un organogélateur, de préférence additif de rhéologie qui comprend au moins un diamide tel que défini ci-haut selon l'invention. Plus particulièrement, ledit organogélateur est un agent ou additif de rhéologie, en particulier agent ou additif thixotrope dans une composition préconcentrée dans un plastifiant organique ou dans un solvant organique sous forme de pâte préactivée.

Est également couvert un organogélateur, de préférence agent ou additif de rhéologie qui comprend au moins un diamide tel que défini ci-haut selon l'invention, plus particulièrement dans une composition préconcentrée dans un plastifiant organique ou dans un solvant organique sous forme de pâte préactivée. La préparation de telles pâtes préactivées préconcentrées peut se faire selon la description de WO 2008/0153924.

Finalement, l'invention couvre une composition de liant organique, laquelle comprend en tant qu'agent de rhéologie au moins un amide tel que défini selon la présente invention. Plus particulièrement, ledit liant organique est un liant pour compositions de revêtements parmi peintures, vernis, encres, enduits gélifiés ou un liant pour compositions de colles ou adhésifs ou un liant pour compositions de mastic ou d'agents d'étanchéité ou d'agent décapant ou pour des compositions de moulage ou enfin une composition de cosmétique. De préférence, ledit liant est sélectionné parmi les résines époxy, polyesters insaturés et saturés, vinyl esters, alkydes, résines silanées, polyuréthanes, polyester-amides, résines acryliques solvantées, c'est-à-dire en milieu solvant organique non réactif, monomères et/ou oligomères acryliques multifonctionnels ou résines acryliques acrylées avec diluant réactif ou les résines inertes diluées dans un solvant réactif ou non réactif. Les résines inertes selon l'invention sont les élastomères chlorés ou non chlorés ou autres polymères non élastomères, chlorés par exemple à base de chlorure de vinyle. Le solvant réactif peut être un monomère dans lequel lesdites résines sont solubles et le solvant non réactif peut être un solvant organique de ladite résine qui est inerte chimiquement.

Les compositions de moulage sont en particulier des compositions de moulage pour composites, y compris composites à renfort fibreux ou des compositions pour pièces moulées, par exemple de type SMC ou BMC ou stratifiées, type coques de bateaux ou panneaux en composites ou pour pièces moulées par coulée ou pour pièces moulées avec application de la composition par projection au pistolet ou à la brosse ou au rouleau.

Ces additifs spécifiques permettent de modifier la viscosité des compositions de mastics, de colles, d'adhésifs, de revêtements tels que les peintures, vernis, enduits gélifiés, encres ou de compositions de moulage ou d'agent d'étanchéité ou de cosmétique.

Les amides selon l'invention se présentent sous forme de poudre micronisée ayant une taille moyenne de particules allant de 5 à 15 µm.

Pour être utilisé comme additif organogélateur et plus particulièrement thixotrope dans une composition d'application telle qu'une composition de revêtement comme une peinture, vernis, enduits gélifiés, encre ou une composition de colle ou d'adhésif ou dans une composition d'agent d'étanchéité ou des mastics ou une composition de moulage ou de cosmétique, ledit amide a besoin d'être activé pour avoir son caractère thixotrope. Selon une première option préférée, ceci peut se faire indépendamment de la composition finale d'application dans une composition préconcentrée dudit amide dans un plastifiant organique ou dans un solvant organique qui est liquide à l'ambiante et adapté à l'amide et à l'application finale et préactivée sous forme de pâte préactivée, comme décrit dans WO 2008/0153924. Dans ce cas, cette composition préactivée d'amide est rajoutée dans la composition d'application finale sans besoin d'activation, dans la mesure où ledit amide est additionné déjà préactivé avec sa composition de pâte « préactivée », « préconcentrée» et adaptée pour l'application finale. Dans ce cas, l'utilisateur final qui est le formulateur n'aura pas besoin d'activer sa formulation dans la mesure où ledit amide préactivé ainsi additionné confère ce caractère dès son mélange dans ladite composition finale d'application.

A défaut de préactivation sous forme de pâte préactivée préconcentrée dans un milieu adapté (compatible) à l'application finale, l'activation dudit amide peut être réalisée, selon une deuxième option, in situ dans la composition d'application finale, mais par l'utilisateur final. L'amide de l'invention peut donc être préactivé sous forme de pâte préactivée et préconcentrée.

Cette activation requiert un cisaillement à haute vitesse et un chauffage correspondant avec des montées de température pouvant aller jusqu'à près de 120°C selon les produits, ainsi qu'une durée minimale nécessaire, dépendante des conditions de température et du système, pour développer des propriétés rhéologiques finales optimales. Ces additifs confèrent à la composition à laquelle ils sont incorporés un comportement thixotrope caractérisé par une rhéofluidification marquée, c'est-à-dire une réduction de la viscosité lorsque le cisaillement augmente, puis une reprise en viscosité dépendante du temps (équivalent à un effet d'hystérésis). Ainsi, ce type d'additifs apporte à la composition finale d'excellentes propriétés d'application qui se caractérisent par une forte viscosité au repos, une bonne stabilité de cette viscosité au stockage, une bonne anti-sédimentation, une facilité d'application et d'extrusion suivant l'application et une bonne résistance à la coulure une fois appliquée.

L'amide d'acide gras de l'invention peut être obtenu par condensation (réaction de condensation) entre au moins une amine primaire selon a), l'hydroxy acide carboxylique gras saturé selon b) et en option en présence d'un acide monocarboxylique selon c), éventuellement en présence d'une deuxième diamine primaire selon d), a), b), c) et d) étant comme définis ci-haut selon l'invention. Le produit de réaction peut optionnellement être dilué dans de l'huile de ricin hydrogénée ou (en option particulière) dans l'huile lesquérolique hydrogénée, et dans ce cas (dilution dans lesdites huiles) à un taux qui peut varier de 10 à 100% en poids par rapport au total amide + huile de ricin hydrogénée ou huile lesquérolique hydrogénée suivant le cas, et de préférence un taux variant de 20 à 100% en poids est utilisé. L'huile de ricin hydrogénée ou l'huile lesquérolique hydrogénée peut être utilisée pour adapter l'affinité du mélange final (amide + huile de ricin hydrogénée ou huile lesquérolique hydrogénée) par rapport à la composition de la formulation finale d'application.

Dans le cas de dilution dans l'huile de ricin hydrogénée ou huile lesquérolique hydrogénée, l'addition se fait à une température comprise entre 140 et 220°C. A la fin de l'addition, on obtient une masse solide qui est broyée sous forme de poudre.

Ledit amide d'acide gras peut donc être utilisé sous forme de poudre ou de pâte préactivée comme décrit ci-haut. La poudre a une granulométrie inférieure à 100 µm et de préférence inférieure à 50 µm et, plus préférentiellement, au moins 90% dudit amide a une granulométrie inférieure à 20 µm et de préférence inférieure à 15 µm.

Les exemples décrits ci-dessous en partie expérimentale sont présentés pour illustrer l'invention et ses performances et ne limitent en rien la portée revendiquée.

### Partie Expérimentale

### I - Matières premières utilisées

**Tableau 1 : Matières premières utilisées**

| Produit | Fonction | Référence commerciale | Fournisseur |
|---|---|---|---|
| Acide 12-hydroxystéarique | Réactif | 12-HSA | Jayant Agro |

| Acide Stéarique | Réactif | Acide Stéarique 99% | VWR |
|---|---|---|---|
| Acide 9 et 10 hydroxy stéarique | Réactif | Voir III-2 | |
| 14-hydroxy-eicosanoïque | Réactif | Voir III-1 | |
| Hexaméthylènediamine | Réactif | Hexaméthylènediamine 98% | Aldrich |
| Acide hexanoïque | Réactif | Acide Hexanoique 99% | Aldrich |
| Ethylène diamine | Réactif | Ethylènediamine ≥ 99,5% (GC) | Aldrich |
| Résine époxy | liant | Araldite® GZ 7071X75 | Huntsman |
| Résine époxy | liant | Araldite® GY 783 BD | Huntsman |
| Agent débullant | Agent débullant | BYK® A530 | Byk |
| Agent Dispersant | Dispersant | Disperbyk® 110 | Byk |
| Dioxyde de titane | Pigment | Tiona® 595 | Société des ocres de France |
| Oxyde de fer | Pigment | Bayferrox® 915 | Lubrizol |
| Phosphate de zinc | Pigment | ZP® 10 | HEUCOPHOS |
| Talc | Additif | Finntalc® MO5 | Mondo minerals |
| Silice | Charge | HPF6 | Sibelco |
| n-Butanol | Solvant | n-Butanol | Aldrich |
| Polyamide | Durcisseur | CRAYAMID® 140 | Arkema |
| Xylène | solvant | Xylène, reagent grade | Aldrich |

### II - Méthodes et tests utilisés

Les formulations sont évaluées avec deux tests : le test de résistance à l'écoulement (ou résistance à la coulure) et une évaluation de la viscosité à différentes vitesses de cisaillement.

### - Test de résistance à l'écoulement

Il s'effectue à l'aide d'un contrôleur de coulure (Levelling/Sagging Tester de Sheen Instruments®) qui permet d'établir la résistance d'un revêtement à la coulure due à la gravité. Fabriqué en inox et doté d'une lame droite, ce contrôleur comporte des encoches de valeurs croissantes.

Le test consiste à déposer des bandes parallèles de peinture d'épaisseurs différentes sur une bande de contraste grâce au contrôleur de coulure. La carte de contraste est immédiatement placée en position verticale, le film le plus fin en haut. L'épaisseur à laquelle les bandes se rejoignent indique la tendance à la coulure (performance de résistance notée).

### - Evaluation de la viscosité

Elle s'effectue ici à l'aide d'un Brookfield® RV à 25°C (mobile : S 4). La vitesse du mobile est fixée à 50 rpm (rotations par minute) et l'on mesure la viscosité de chaque peinture une fois cette viscosité stabilisée. On répète l'opération pour une vitesse de 20 rpm, 10 rpm, 5 rpm, 1 rpm.

### III - Préparation et caractérisation des organogélateurs et d'additifs de rhéologie

### III -1 Préparation du 14-HEA

### A1) Préparation des acides gras hydrogénés de Lesquerella à partir d'huile de Lesquerella

### Huile de Lesquerella : mode d'obtention à partir de graines de Lesquerella

Le mode opératoire sur 25 kg de graines (Technology Crops International) est le suivant :
1. Floconnage de la graine de Lesquerella fraîche sur aplatisseur à rouleaux lisses.
2. Les flocons sont ensuite séchés 16 h à 100°C.
3. Les flocons sont introduits dans une colonne de percolation.
4. Le mélange méthanol/hexane (50/50 en poids) est alors circulé sur le lit de flocons pendant 30 minutes à 40°C.
5. Le miscella est ensuite soutiré et le lit de flocons est ensuite lavé par 5 lavages successifs avec le mélange méthanol/hexane à 40°C (5 minutes par lavage).
6. Le miscella est alors évaporé sous vide à 90°C sous 20 mbars pendant 5 min.
7. L'huile et les gommes sont séparées par centrifugation. Le rendement en huile est calculé sur la base de la masse d'huile obtenue par rapport au poids d'huile théorique attendu.
8. L'huile est ensuite lavée jusqu'à neutralité par ajout d'eau chaude et centrifugation, puis elle est séchée sous vide à 90°C et 20 mbars pendant 5 min. L'indice d'acide et la composition de cette huile sont alors mesurés.

**Tableau 2 : Analyse de l'huile extraite avec un mélange méthanol/hexane**

| Critères | Méthode | |
|---|---|---|
| Indice d'acide (mg KOH/g) | EN 14104 | 11,2 |
| Profil en acides gras | EN14105 | |
| Palmitique (C16:0) | | 1,6 |
| Palmitoléique (C16:1) | | 0,9 |
| Stéarique (C18:0) | | 2,3 |
| Oléique (C18:1) | | 18,8 |
| Ricinoléique (C18:1-OH) | | 0,5 |
| Linoléique (C18:2) | | 10,1 |
| Densipoléique (18:2-OH) | | 0,2 |
| Linolénique et Arachidique | | 11,6 (1) |
| Eicosénoïque (C20:1) | | 0,9 |
| Lesquérolique (C20:1-OH) | | 52,1 |
| Auricolique (20:2-OH) | | 2,6 |
| Phospholipides (%) | Interne | 1,3 (2) |
| Rendement corrigé en huile (3), % | - | 106,5 |

| | | |
|---|---|---|
| (1) Les deux pics sont co-élués. L'acide linolénique est majoritaire (2) Valeur calculée ; % phospholipides = % phosphore x 26 (3) Rdt corrigé = Rdt Huile d'extraction - % phospholipides | | |

L'huile ainsi obtenue est ensuite raffinée par neutralisation à la soude et dégommage à l'acide phosphorique dilué de façon à éliminer les phospholipides. Finalement, l'huile est séchée sous vide. L'huile obtenue a les caractéristiques suivantes :
Indice d'acide : 0,5 mg KOH/g
Indice de saponification : 175 mg KOH/g
Indice d'hydroxyle : 100 mg KOH/g
Indice d'iode : 95 g I₂/100 g
Teneur en acide lesquérolique : 52%
Teneur en phosphore : 10 ppm
Teneur en eau et volatils : 0,1% pds
Teneur en cendres : 0,1% pds.

### Transestérification de l'huile au méthanol

Dans un premier temps, on procède à une transestérification (au méthanol) de l'huile de Lesquerella, puis à une hydrogénation et finalement à une hydrolyse. Une étape d'extraction après la transestérification permet d'enrichir le produit en ester d'acide lesquérolique.

La méthanolyse de l'huile de Lesquerella est effectuée avec un rapport molaire méthanol/huile de 6 (soit deux fois la quantité stoechiométrique). Le catalyseur utilisé est le méthylate de sodium à une teneur de 0,5% poids et la température de la réaction est de 60°C. Les constituants sont mélangés sous forte agitation pendant 30 minutes. Après méthanolyse (transestérification) et élimination de la glycérine par décantation, les esters sont purifiés par lavage à l'eau et séchage sous vide. Les spécifications des esters méthyliques sont les suivantes :
Indice d'acide : 0,5 mg KOH/g
Indice de saponification : 175 mg KOH/g
Indice d'iode : 95 g I₂/100 g
Teneur en glycérides résiduels (analyse par CPG) : 1,9% pds
Teneur en acide lesquérolique (ester méthylique) : 52%

### A2) Préparation d'un mélange enrichi en acide 14-hydroxy-eicosanoïque à partir d'esters méthyliques comme décrit dans le paragraphe précédent A1)

Le mélange d'esters issus de l'étape de transestérification est soumis à une étape d'extraction liquide-liquide avec un mélange méthanol/hexane. Dans la réalisation pratique de l'exemple, le méthanol contient 5% en poids d'eau. Les acides gras non hydroxylés sont plus compatibles avec la phase hexane, alors que les acides gras hydroxylés comme l'acide lesquérolique sont plus compatibles avec la phase méthanolique. L'hexane a été utilisé en tant que solvant apolaire et le solvant polaire est constitué de méthanol hydraté. On procède à une succession d'étapes d'appauvrissement et d'enrichissement.
1. 5 g (ester méthylique d'huile de Lesquerella) + 30 mL de solvant apolaire + 15 mL de solvant polaire sont agités pendant 5 minutes dans une ampoule à décanter et donnent une phase lourde PL1 + phase légère pl1.
2. La phase légère pl1 est reprise avec 15 mL de solvant polaire et donne à nouveau une phase lourde PL2 et une phase légère pl2.
3. La phase lourde PL1 et la phase lourde PL2 sont reprises avec 30 mL de solvant apolaire et donnent à nouveau une phase lourde PL3 et une phase légère pl3.
4. La phase lourde PL3 est reprise avec 30 mL de solvant apolaire pour donner une phase lourde PL4 et une légère pl4.

On concentre ensuite, par évaporation des solvants, les fractions récupérées.
1. La phase lourde PL4 donne la fraction polaire.
2. Les phases légères pl2 + pl3 + pl4 sont combinées pour donner la fraction apolaire.

**Tableau 3 : Bilan analytique des esters issus de l'extraction**

| | Matière première | Phase Lourde | Phase Légère |
|---|---|---|---|
| Solvant polaire | | Méthanol 95% | Méthanol 95% |
| Solvant apolaire | | Hexane | Hexane |
| Rdt* massique, % | | 16,5 | 83,5 |
| Rdt* extraction Lesquerolate de méthyle, % | | 25,4 | 74,8 |
| Indice d'acide | 0,72 | nd | 1,12 |
| Me C16:1 (%) | 0,5 | 0,1 | 0,6 |
| Me C16 (%) | 1,3 | 0,1 | 1,6 |
| Me C18 :2 (%) | 9,1 | 0,6 | 11,7 |
| Me C18 :1 (%) | 23,5 | 0,3 | 27,5 |
| Me C18 :0 (%) | 1,8 | 0,1 | 2,2 |
| Me C20:0 (%) | 0,9 | 0,0 | 1,1 |
| Me C20:1 (%) | 1,0 | 0,0 | 0,7 |
| Me C18 :1-OH (%) | 0,3 | 0,2 | 0,3 |
| Me C20 :1-OH (%) | 60,3 | 92,9 | 54,0 |
| MonoGlycéride (%) | 1,7 | 5,8 | 0,8 |
| DiGlycéride (%) | 0,1 | 0,0 | 0,1 |
| TriGlycéride (%) | 0,0 | 0,0 | 0,0 |

| | | | |
|---|---|---|---|
| Rdt : rendement | | | |

### Hydrogénation de l'ester méthylique enrichi

Sur la fraction enrichie en acide lesquérolique (sous forme d'ester Me), on procède à une hydrogénation et à une hydrolyse pour obtenir un mélange riche en acide 14-hydroxy-eicosanoïque (hydrogénation et hydrolyse décrites ci-dessous).

Pour l'hydrogénation en autoclave, on utilise un catalyseur de type nickel de Raney fourni par Johnson Matthey, à une teneur de 0,5% en poids. La température d'hydrogénation est de 150°C sous une pression d'hydrogène de 8 bars. Cette étape conduit à un produit ayant un indice d'iode de 3 g I₂/100 g.

### Saponification de l'ester méthylique hydrogéné

On procède finalement à une étape de saponification (hydrolyse) par ajout de soude, puis à une étape d'acidification à l'acide sulfurique. Le mélange résultant est lavé à l'eau, décanté et séché sous vide.

Les caractéristiques du mélange obtenu sont :
Indice d'acide : 1 mg KOH/g
Indice d'hydroxyle : 145 mg KOH/g
Indice d'iode : 3 g I₂/100 g
Teneur en acide 14-hydroxy-eicosanoïque : 89%.

### III-2 Préparation d'un mélange d'acides hydroxy-9 et hydroxy-10 stéariques (9-HSA et 10-HSA)

La préparation est basée sur l'hydroxylation de la double liaison d'un acide gras (transposée ici à l'acide oléique) comme décrite dans « Addition of Formic acid to Oleifinic compounds » de HB Knights, R. E Koos and Daniel Swern, 2 Mai 1953.

D'autres méthodes peuvent être utilisées pour accéder aux acides gras monohydroxylés 9- et 10-HSA.

### Méthode de préparation utilisée

Dans un ballon de 1 litre équipé d'un thermomètre, d'un Dean-Stark, d'un condensateur et d'un agitateur, on introduit sous une atmosphère d'azote, 319,1 grammes d'acide oléique, 677,6 grammes d'acide formique et 3,3 grammes d'acide perchlorique. Après 30 minutes à reflux, l'acide formique en excès est évaporé sous vide à 75 mbar et 65°C.

Le composé obtenu (102 grammes) est ensuite hydrolysé par une solution de soude 6N (100 grammes). Enfin, le produit est neutralisé par addition lente d'acide chlorhydrique fumant (64 grammes) dans 66 grammes d'eau.

La purification se fait par solubilisation du milieu réactionnel dans du toluène et par trois lavages successifs à l'aide d'une solution de NaCl 11%. Le toluène est ensuite évaporé et le produit est recristallisé dans l'hexane. 27 grammes d'un mélange d'acides gras monohydroxylés 9- et -10 HSA sont ainsi obtenus.

### III - 3 Préparation d'amides selon l'invention et comparatifs

### EXEMPLE 1 : Diamide A comparatif (basé sur l'acide stéarique)

Dans un ballon de 1 litre équipé d'un thermomètre, d'un Dean-Stark, d'un condensateur et d'un agitateur, on introduit dans une atmosphère d'azote, 49,96 grammes d'hexaméthylène diamine (soit 0,43 moles, 0,86 équivalent amine), 244,65 grammes (0,86 moles, 0,86 équivalent) d'acide stéarique.

Le mélange est chauffé à 200°C toujours sous courant d'azote. L'eau éliminée commence à s'accumuler dans le Dean Stark dès 150°C. La réaction est contrôlée par l'indice d'acide et d'amine. Lorsque les valeurs (indices) d'acides et d'amines sont inférieures à 10 mg KOH/g, le mélange réactionnel est refroidi à 150°C, puis déchargé dans un moule siliconé. Une fois refroidi à la température ambiante, le produit est micronisé mécaniquement par broyage et tamisage pour obtenir une granulométrie fine et contrôlée avec taille moyenne obtenue de 7 µm.

### EXEMPLE 2 : Diamide B Comparatif (basé sur les acides hydroxy-9 et -10 stéariques)

Dans un ballon de 1 litre équipé d'un thermomètre, d'un Dean Stark, d'un condensateur et d'un agitateur, on introduit dans une atmosphère d'azote, 49,96 grammes d'hexaméthylène diamine (soit 0,43 moles, 0,86 équivalent amine), 260,48 grammes de mélange d'acides hydroxy-9 et -10 stéariques (soit 0,86 moles, 0,86 équivalent acide) comme décrit dans le paragraphe III-2. Le mélange est chauffé à 200°C toujours sous courant d'azote. L'eau éliminée s'accumule dans le Dean Stark dès 150°C. La réaction est contrôlée par l'indice d'acide et d'amine. Lorsque les valeurs d'acide et d'amine sont inférieures à 10 mg KOH/g, le mélange réactionnel est refroidi à 150°C, puis déchargé dans un moule siliconé. Une fois refroidi à la température ambiante, le produit est micronisé mécaniquement comme dans l'exemple 1 avec même taille moyenne.

### EXEMPLE 3 : Diamide C comparatif (basé sur le 12 HSA)

Dans un ballon de 1 litre équipé d'un thermomètre, d'un Dean Stark, d'un condensateur et d'un agitateur, on introduit dans une atmosphère d'azote, 49,96 grammes d'hexaméthylène diamine (soit 0,43 moles, 0,86 équivalent amine), 271,04 grammes de mélange d'acides hydroxy-12 stéarique (soit 0,86 moles, 0,86 équivalent acide). Le mélange est chauffé à 200°C toujours sous courant d'azote. L'eau éliminée s'accumule dans le Dean Stark dès 150°C. La réaction est contrôlée par l'indice d'acide et d'amine. Lorsque les valeurs d'acide et d'amine sont inférieures à 10 mg KOH/g, le mélange réactionnel est refroidi à 150°C, puis déchargé dans un moule siliconé. Une fois refroidi à la température ambiante, le produit est micronisé mécaniquement comme dans l'exemple 1 avec même taille moyenne.

### EXEMPLE 4 : Diamide D selon l'invention

Dans un ballon de 1 litre équipé d'un thermomètre, d'un Dean Stark, d'un condensateur et d'un agitateur, on introduit dans une atmosphère d'azote, 49,96 grammes d'hexaméthylène diamine (soit 0,43 moles, 0,86 équivalent amine), 307,30 grammes de mélange enrichi en acide 14-hydroxy-eicosanoïque (soit 0,86 moles, 0,86 équivalent acide) comme décrit ci haut (produit final A2 à 89% de 14-HEA). Le mélange est chauffé à 200°C toujours sous courant d'azote. L'eau éliminée s'accumule dans le Dean Stark dès 150°C. La réaction est contrôlée par l'indice d'acide et d'amine. Lorsque les valeurs d'acide et d'amine sont inférieures à 10 mg KOH/g, le mélange réactionnel est refroidi à 150°C, puis déchargé dans un moule siliconé. Une fois refroidi à la température ambiante, le produit est micronisé mécaniquement comme dans l'exemple 1 avec même taille moyenne.

### EXEMPLES 5, 6, 7 et 8 : Diamide E comparatif (ex. 5), Diamide F comparatif (ex. 6), Diamide G comparatif (ex. 7), Diamide H selon l'invention (ex. 8).

Le même mode opératoire a été utilisé que dans les exemples 1 à 4 mais avec les composants de réaction suivants présentés dans le tableau ci-dessous :

**Tableau 4 : Composition des diamides comparés**

| **Exemple** | **Réactif** | **Moles** | **Equivalents** |
|---|---|---|---|
| 1 | L'hexaméthylène | 0,43 | 0,86 |
| Diamide A | diamine | | |
| comparatif | Acide stéarique | 0,86 | 0,86 |
| 2 | L'hexaméthylène | 0,43 | 0,86 |
| Diamide B | diamine | | |
| comparatif | Acides hydroxy-9 et -10 stéariques | 0,86 | 0,86 |
| 3 | L'hexaméthylène | 0,43 | 0,86 |
| Diamide C | diamine | | |
| comparatif | Acide 12-hydroxy-stéarique | 0,86 | 0,86 |
| 4 | L'hexaméthylène | 0,43 | 0,86 |
| Diamide D selon l'invention | diamine | | |
| | Acides 14 hydroxy-eicosanoïque | 0,86 | 0,86 |
| 5 | L'éthylène diamine | 0,5 | 1 |
| Diamide E | Acide Hexanoïque | 0,5 | 0,5 |
| comparatif | Acide stéarique | 0,5 | 0,5 |
| 6 | L'éthylène diamine | 0,5 | 1 |
| Diamide F | Acide Hexanoïque | 0,5 | 0,5 |
| comparatif | Acides hydroxy-9 et -10 stéariques | 0,5 | 0,5 |
| 7 | L'éthylène diamine | 0,5 | 1 |
| Diamide G | Acide Hexanoïque | 0,5 | 0,5 |
| comparatif | Acide 12-hydroxy-stéarique | 0,5 | 0,5 |
| 8 | L'éthylène diamine | 0,5 | 1 |
| Diamide H selon l'invention | Acide Hexanoïque | 0,5 | 0,5 |
| | Acides 14 hydroxy-eicosanoïque | 0,5 | 0,5 |

### III - 4 Propriétés d'organogélateur pour diamides A, B, C, D

20 grammes de diamide A de l'exemple 1 (comparatif) préalablement broyé (Taille moyenne, d50 = 10 µm) et 80 grammes de Xylène sont chargés dans une boite métallique à température ambiante. En utilisant un disperseur type Dispermat® CV muni d'une pâle de 4 cm de diamètre, les deux produits sont mélangés à une vitesse de 2000 tours/min (ou rpm) pendant 30 minutes à une température n'excédant pas 20°C, par régulation de la température par circulation d'eau froide.

Activation : La boite est ensuite refermée soigneusement et introduite dans une étuve préalablement préchauffée à 65°C pendant 24 heures. Une fois refroidi et après 4 heures de repos, l'aspect du mélange est observé. La même procédure est réalisée sur les diamides des exemples 2 (B comparatif), 3 (C comparatif), 4 (D selon l'invention). Les résultats sont présentés dans le tableau 5.

**Tableau 5 : Aspect des mélanges après l'activation**

| **Amide selon exemple** | **Aspect** |
|---|---|
| 1 (A) | Liquide |
| 2 (B) | Pâte molle mais permettant à une spatule de bois de rester à la verticale |
| 3 (C) | Pâte dure permettant à une spatule métallique de rester à la verticale |
| 4 (D) | Pâte dure permettant à une spatule métallique de rester à la verticale. Consistance plus importante que C de l'exemple 3. |

Les résultats montrent que l'invention se présente, après l'étape d'activation et de refroidissement, sous forme de pâte dure permettant à une spatule métallique de rester à la verticale dans la pâte. Ce résultat illustre parfaitement la capacité du diamide D de l'exemple 4 selon l'invention à être un organogélateur contrairement à l'amide A de l'exemple 1. De plus, la consistance du gel est bien meilleure avec l'amide D de l'exemple 4 à base de 14- HEA que l'amide B de l'exemple 2 à base de 9- et 10- HSA et d'être au moins aussi bon que l'amide C de l'exemple 3 à base du 12- HSA comme visé au départ.

### IV- Evaluation des performances rhéologiques dans une formulation de peinture

### Formulations de peintures utilisées pour l'évaluation

### 1 - Préparation

Une formulation dite « millbase » est préparée avec les proportions du tableau 3 de la manière suivante :
Dans un bol de disperseur (Dispermill 2075 yellow line, fournisseur : Erichsen®) chauffé par un système de double enveloppe :
1. Introduction des liants époxy ainsi que le dispersant et l'agent débullant. L'homogénéisation se fait après 2 minutes à 800 tours/minute.
2. Introduction des charges et pigments, puis broyage à 3000 tours/minute pendant 30 minutes à l'aide d'une pâle de 7 cm. Grâce au bol à double enveloppe, cette étape est refroidie avec un bain d'eau froide (20°C).
3. Introduction des solvants.

### 2 - Activation

24 heures après la préparation de la millbase, la formulation est de nouveau dispersée à 3000 tours/minute à l'aide d'une pâle de 4 cm. Le diamide à tester est introduit dans la millbase à une température d'activation donnée (variant de 40°C à 70°C) pendant 20 minutes à 3000 tours/minute.

Après l'ajout du durcisseur dilué dans la millbase, les peintures sont ajustées avec un mélange xylène/butanol (1/1) à une viscosité d'utilisation de 0,4 Pa.s, mesurée sur le cône 4 à 25°C à 2500 s⁻¹ à l'aide du Brookfield® CAP 1000. Les proportions entre le durcisseur et le mélange de solvants sont définies dans le tableau 6.

Après l'ajustement, la peinture est mélangée à 1500 tours/minute durant 2 minutes, puis laissée au repos pendant 30 minutes.

**Tableau 6 : formulation « Millbase »**

| **Composition de la Millbase** | **Fonction** | **% massique** |
|---|---|---|
| Araldite® GZ 7071X75 | liant | 17,3 |
| Araldite® GY 783 BD | liant | 12,9 |
| BYK® A530 | Agent débullant | 0,5 |
| Disperbyk® 110 | Dispersant | 0,5 |
| Tiona 595 (Titanium dioxide) | Pigment | 1,9 |
| Bayferrox® 915 595 (oxide de fer) | Pigment | 4,1 |
| ZP® 10 (phosphate de zinc) | Pigment | 7,5 |
| Finntalc® MO5 | charge | 9,4 |
| Silice HPF6 | charge | 19,0 |
| n-Butanol | Solvant | 5,4 |
| Diamide | Additif de Rhéologie | 0,8 |
| **TOTAL** | | **79,3** |

**Tableau 7 : durcisseur**

| **Composition du durcisseur** | **% massique** |
|---|---|
| CRAYAMID® 140 | 8,8 |
| Xylène | 11,9 |
| **TOTAL** | **20,7** |

### 3 - Evaluation de la rhéologie des formulations et résultats (voir tableaux 8 et 9)

Différentes formulations de peinture ont été réalisées suivant les proportions du tableau 3 et 4 et avec différentes températures d'activation allant de 40 à 70°C suivant le protocole énoncé plus haut.

Les résultats de résistance à la coulure et de rhéologie montrent que le diamide H de l'invention à base de 14-HEA a :
- une résistance à la coulure (tableau 8) meilleure que le diamide F à base de 9- et 10-HSA et aussi performant que le diamide à base de 12-HSA à une température d'activation de 70°C dans cette formulation. Il présente donc les caractéristiques nécessaires d'un additif de rhéologie.
- un effet thixotropique sur la formulation une fois qu'il est activé au-delà de 40°C contrairement au diamide E (à base d'acide stéarique) qui est inactif quelle que soit la température (tableau 9) et le diamide H selon l'invention est meilleur que le diamide F à base d'acide 9- et 10- hydroxy stéarique.

**Tableau 8 : résultats de résistance à la coulure**

| **Essai** | **Diamide** | **Résistance à la coulure (µm)** |
|---|---|---|
| **Exemple 5, 40°C** | E | 225-250 |
| **Exemple 5, 50°C** | E | 225-250 |
| **Exemple 5, 60°C** | E | 225-250 |
| **Exemple 5, 70°C** | E | 225-250 |
| **Exemple 6, 40°C** | F | 225-250 |
| **Exemple 6, 50°C** | F | 225-250 |
| **Exemple 6, 60°C** | F | 375-400 |
| **Exemple 6, 70°C** | F | 400-425 |
| **Exemple 7, 40°C** | G | 550-575 |
| **Exemple 7, 55°C** | G | 550-575 |
| **Exemple 7, 70°C** | G | > 750 |
| **Exemple 7, 80°C** | G | > 750 |
| **Exemple 8, 40°C** | H | 400-425 |
| **Exemple 8, 55°C** | H | 400-425 |
| **Exemple 8, 70°C** | H | > 750 |
| **Exemple 8, 80°C** | H | > 750 |

**Tableau 9 : résultats rhéologiques**

| **Conditions d'essai** | **Diamide** | **Viscosité Brookfield à 25°C (mPa.s)** | | | | |
|---|---|---|---|---|---|---|
| | | **1 RPM** | **5 RPM** | **10 RPM** | **50 RPM** | **100 RPM** |
| **Exemple 4, 40°C** | E | 2000 | 1150 | 906 | 657 | 588 |
| **Exemple 5, 50°C** | E | 1912 | 1002 | 858 | 642 | 580 |
| **Exemple 5, 60°C** | E | 1970 | 1055 | 880 | 640 | 584 |
| **Exemple 5, 70°C** | E | 1989 | 1107 | 900 | 654 | 588 |
| **Exemple 6, 40°C** | F | 2000 | 1120 | 920 | 660 | 596 |
| **Exemple 6, 50°C** | F | 2400 | 1280 | 1020 | 700 | 630 |
| **Exemple 6, 60°C** | F | 9600 | 3400 | 2300 | 1092 | 862 |
| **Exemple 6, 70°C** | F | 11800 | 4000 | 2640 | 1196 | 922 |
| **Exemple 7, 40°C** | G | 17600 | 5360 | 3420 | 1456 | 1076 |
| **Exemple 7, 55°C** | G | 18200 | 5600 | 3600 | 1528 | 1128 |
| **Exemple 7, 70°C** | G | 30400 | 8680 | 5280 | 2040 | 1426 |
| **Exemple 7, 80°C** | G | 30800 | 8360 | 5360 | 2064 | 1456 |
| **Exemple 8, 40°C** | H | 8600 | 2880 | 1960 | 972 | 772 |
| **Exemple 8, 55°C** | H | 9800 | 3280 | 2240 | 1064 | 832 |
| **Exemple 8, 70°C** | H | 22000 | 6480 | 4060 | 1644 | 1182 |
| **Exemple 8, 80°C** | H | 23000 | 6480 | 4280 | 1776 | 1280 |

## Revendications

1. Amide d'acide gras, **caractérisé en ce qu'**il comprend au moins un produit de réaction d'un mélange réactionnel comprenant :
a) au moins une amine sélectionnée parmi :
▪ une amine aliphatique linéaire, en C₂ à C₁₂, et/ou
▪ une amine cycloaliphatique en C₆ à C₁₈, et/ou
▪ une amine aromatique,
b) l'acide 14-hydroxy-eicosanoïque en l'absence ou en présence d'acide 12-hydroxy-stéarique,
c) en option, au moins un monoacide sélectionné parmi les acides carboxyliques linéaires saturés et non hydroxylés en C₆ à C₁₈,
d) en option au moins une deuxième amine différente de a), en particulier une diamine, sélectionnée parmi les amines aliphatiques linéaires en C₂ à C₁₂,
et **en ce que** ledit amide est un diamide et que lesdites amines a) et d) sont des diamines.

2. Amide selon la revendication 1, **caractérisé en ce que** ledit acide 12-hydroxy-stéarique est présent et **en ce que** le taux molaire de l'acide 14-hydroxy-eicosanoïque varie de 10 à 99%, dudit composant b).

3. Amide selon l'une des revendications 1 ou 2, **caractérisé en ce que** ledit composant b) est un mélange d'acide 14-hydroxy-eicosanoïque et d'acide 12-hydroxy-stéarique.

4. Amide selon l'une des revendications 1 ou 2, **caractérisé en ce que** ledit acide 12-hydroxy-stéarique est absent dudit composant b) et qu'il est remplacé totalement par l'acide 14-hydroxy-eicosanoïque.

5. Amide selon l'une des revendications 1 à 3, **caractérisé en ce que** ledit diamide porte 2 fonctions amides à base du même hydroxyacide b) quand 12-HSA est absent ou à base de deux hydroxyacides b) différents, 14-HEA et 12-HSA, quand le 12-HSA est présent.

6. Amide selon l'une des revendications 1 à 5, **caractérisé en ce que** dans ledit diamide, ledit monoacide c) est présent à un taux tel que le rapport molaire dudit hydroxyacide b) sur ledit monoacide c) est de 1/2 à 4/1.

7. Amide selon l'une des revendications 1 à 6, **caractérisé en ce que** ledit diamide porte une fonction amide à base d'un hydroxyacide selon b) et une autre fonction à base dudit monoacide c) correspondant à un rapport molaire b)/c) de 1/1.

8. Amide selon l'une des revendications 1 à 7, **caractérisé en ce qu'**il comprend au moins 2, de préférence au moins 3 produits de réaction différents, tels qu'issus de la réaction dans ledit mélange réactionnel d'une amine a) ou d) avec un monoacide b) ou c).

9. Amide selon la revendication 8, **caractérisé en ce qu'**il est un diamide et qu'il comprend un mélange de produits de formules suivantes :
- b1-a1-c1
- b1-a1-b1
et de préférence :
- b1-a1-c1
- b1-a1-b1
- b1-a2-b1
avec a1 : résidu de diamine selon a) portant deux groupements amides -NHC(=O)-, a2 : résidu de diamine selon d) portant deux groupements -NHC(=O)-, b1 : résidu de l'acide 14-HEA sans groupement carboxy et c1 : résidu de monoacide non hydroxylé selon c) sans groupement carboxy.

10. Utilisation de l'amide, tel que défini selon l'une des revendications 1 à 9, **caractérisée en ce qu'**il s'agit d'une utilisation en tant qu'organogélateur.

11. Utilisation selon la revendication 10, **caractérisée en ce que** ledit amide est utilisé dans des compositions de revêtements, de colles ou adhésifs, de moulage, de mastics ou d'agents d'étanchéité ou de cosmétique.

12. Organogélateur, **caractérisé en ce qu'**il comprend au moins un diamide tel que défini selon l'une des revendications 1 à 9.

13. Organogélateur selon la revendication 12, **caractérisé en ce qu'**il s'agit d'un agent ou additif de rhéologie thixotrope dans une composition préconcentrée dans un plastifiant organique ou dans un solvant organique, sous forme de pâte préactivée.

14. Composition de liant organique, **caractérisée en ce qu'**elle comprend en tant qu'agent de rhéologie au moins un amide tel que défini selon l'une des revendications 1 à 9.

15. Composition de liant organique selon la revendication 14, **caractérisée en ce que** ledit liant organique est un liant pour compositions de revêtements parmi peintures, vernis, encres, enduits gélifiés ou un liant pour compositions de colles ou d'adhésifs ou un liant pour compositions de mastic ou d'agents d'étanchéité ou d'agent décapant ou pour des compositions de moulage ou des compositions de cosmétique.

16. Composition selon la revendication 14 ou 15, **caractérisée en ce que** ledit liant est sélectionné parmi : résines époxy, polyesters insaturés et saturés, vinyl esters, alkydes, résines silanées, polyuréthanes, polyesters-amides, résines acryliques solvantées, monomères et/ou oligomères acryliques multifonctionnels ou résines acryliques acrylées avec diluant réactif ou les résines inertes diluées dans un solvant réactif ou non réactif.

## Patentansprüche

1. Fettsäureamid, **dadurch gekennzeichnet, dass** es mindestens ein Reaktionsprodukt eines Reaktionsgemisches enthält, umfassend:
a) mindestens ein Amin, ausgewählt aus:
▪ einem linearen aliphatischen C₂- bis C₁₂-Amin, und/oder
▪ einem cycloaliphatischen C₆- bis C₁₈-Amin, und/oder
▪ einem aromatischen Amin,
b) 14-Hydroxyarachdinsäure in Abwesenheit oder in Gegenwart von 12-Hydroxystearinsäure,
c) gegebenenfalls mindestens eine Monosäure, ausgewählt aus gesättigten und nicht hydroxylierten linearen C₆- bis C₁₈-Carbonsäuren,
d) gegebenenfalls mindestens ein zweites Amin, das von a) verschieden ist, insbesondere ein Diamin, ausgewählt aus linearen aliphatischen C₂- bis C₁₂-Aminen,
und dadurch, dass das Amid ein Diamid ist und dass die Amine a) und d) Diamine sind.

2. Amid nach Anspruch 1, **dadurch gekennzeichnet, dass** die 12-Hydroxystearinsäure vorhanden ist und dass das Molverhältnis von 14-Hydroxyarachinsäure zur Komponente b) im Bereich von 10 bis 99 % liegt.

3. Amid nach einem der Ansprüche 1 oder 2, **dadurch gekennzeichnet, dass** die Komponente b) ein Gemisch aus 14-Hydroxyarachinsäure und 12-Hydroxystearinsäure ist.

4. Amid nach einem der Ansprüche 1 oder 2, **dadurch gekennzeichnet, dass** die 12-Hydroxystearinsäure in der Komponente b) nicht vorhanden ist und vollständig durch 14-Hydroxyarachinsäure ersetzt ist.

5. Amid nach einem der Ansprüche 1 bis 3, **dadurch gekennzeichnet, dass** das Diamid 2 Amidfunktionen trägt, basierend auf der gleichen Hydroxysäure b), wenn 12-HSA nicht vorhanden ist, oder basierend auf zwei verschiedenen Hydroxysäuren b), 14-HEA und 12-HSA, wenn 12-HSA vorhanden ist.

6. Amid nach einem der Ansprüche 1 bis 5, **dadurch gekennzeichnet, dass** die Monosäure c) in dem Diamid in einem Anteil vorhanden ist, dass das Molverhältnis der Hydroxysäure b) zur Monosäure c) 1/2 bis 4/1 beträgt.

7. Amid nach einem der Ansprüche 1 bis 6, **dadurch gekennzeichnet, dass** das Diamid eine Amidfunktion basierend auf einer Hydroxysäure gemäß b) und eine weitere Funktion basierend auf der Monosäure c) trägt, was einem Molverhältnis b)/c) von 1/1 entspricht.

8. Amid nach einem der Ansprüche 1 bis 7, **dadurch gekennzeichnet, dass** es mindestens 2, vorzugsweise mindestens 3 verschiedene Reaktionsprodukte enthält, die sich aus der Umsetzung eines Amins a) oder d) mit einer Monosäure b) oder c) in dem Reaktionsgemisch ergeben.

9. Amid nach Anspruch 8, **dadurch gekennzeichnet, dass** es ein Diamid umfasst und dass es ein Gemisch von Produkten mit den folgenden Formeln umfasst:
- b1-a1-c1
- b1-a1-b1
und vorzugsweise:
- b1-a1-c1
- b1-a1 -b1
- b1-a2-b1
wobei a1: Diaminrest nach a), der zwei Amidgruppen -NHC(=O)- trägt, a2: Diaminrest nach d), der zwei - NHC(=O)-Gruppen trägt, b1: Rest der Säure 14-HEA ohne Carboxygruppe und c1: Rest von nicht-hydroxylierter Monosäure gemäß c) ohne Carboxygruppe.

10. Verwendung des Amids nach einem der Ansprüche 1 bis 9, **dadurch gekennzeichnet, dass** es sich um eine Verwendung als organischem Gelbildner handelt.

11. Verwendung nach Anspruch 10, **dadurch gekennzeichnet, dass** das Amid in Zusammensetzungen für Anstriche, Kleber oder Haftmittel, für Formmassen, für Kitt oder Dichtstoffe oder Kosmetik verwendet wird.

12. Organischer Gelbildner, **dadurch gekennzeichnet, dass** er mindestens ein Diamid nach einem der Ansprüche 1 bis 9 umfasst.

13. Organischer Gelbildner nach Anspruch 12, **dadurch gekennzeichnet, dass** es sich um ein thixotropes Rheologieadditiv oder -mittel in einer vorkonzentrierten Zusammensetzung in einem organischen Weichmacher oder in einem organischen Lösemittel in Form einer voraktivierten Paste handelt.

14. Organische Bindemittelzusammensetzung, **dadurch gekennzeichnet, dass** sie als Rheologiemittel mindestens ein Amid nach einem der Ansprüche 1 bis 9 umfasst.

15. Organische Bindemittelzusammensetzung nach Anspruch 14, **dadurch gekennzeichnet, dass** das organische Bindemittel ein Bindemittel für Beschichtungszusammensetzungen aus Farben, Lacken, Tinten, Gelcoats ist, oder ein Bindemittel für Klebstoff- oder Haftmittelzusammensetzungen oder ein Bindemittel für Kitt- oder Dichtmittel- oder Beizzusammensetzungen oder für Formmassenzusammensetzungen oder kosmetische Zusammensetzungen.

16. Zusammensetzung nach Anspruch 14 oder 15, **dadurch gekennzeichnet, dass** das Bindemittel ausgewählt ist aus: Epoxidharzen, ungesättigten und gesättigten Polyestern, Vinylestern, Alkyden, Silanharzen, Polyurethanen, Polyesteramiden, Acrylharzen auf Lösungsmittelbasis, multifunktionellen Acrylmonomeren und/oder -oligomeren oder mit reaktivem Verdünnungsmittel acrylierten Acrylatharzen oder in einem reaktiven oder nicht-reaktiven Lösungsmittel verdünnten inerten Harzen.

## Claims

1. Fatty acid amide, **characterized in that** it comprises at least one product of reaction of a reaction mixture comprising:
a) at least one amine selected from:
▪ a C₂ to C₁₂ linear aliphatic amine, and/or
▪ a C₆ to C₁₈ cycloaliphatic amine, and/or
▪ an aromatic amine,
b) 14-hydroxyeicosanoic acid in the absence or in the presence of 12-hydroxystearic acid,
c) optionally, at least one monoacid selected from C₆ to C₁₈ non-hydroxylated saturated linear carboxylic acids,
d) optionally, at least a second amine different from a), which may in particular be a diamine, selected from C₂ to C₁₂ linear aliphatic amines,
**in that** said amide is a diamide and said amines a) and d) are diamines.

2. Amide according to Claim 1, **characterized in that** said 12-hydroxystearic acid is present and **in that** the molar content of 14-hydroxyeicosanoic acid ranges from 10% to 99% of said component b).

3. Amide according to either of Claims 1 and 2, **characterized in that** said component b) is a mixture of 14-hydroxyeicosanoic acid and 12-hydroxystearic acid.

4. Amide according to either of Claims 1 and 2, **characterized in that** said 12-hydroxystearic acid is absent from said component b) and **in that** it is totally replaced with 14-hydroxyeicosanoic acid.

5. Amide according to one of Claims 1 to 3, **characterized in that** said diamide bears two amide functions based on the same hydroxy acid b) when 12-HSA is absent or based on two different hydroxy acids b), 14-HEA and 12-HSA, when 12-HSA is present.

6. Amide according to one of Claims 1 to 5, **characterized in that**, in said diamide, said monoacid c) is present in a content such that the mole ratio of said hydroxy acid b) to said monoacid c) is from 1/2 to 4/1.

7. Amide according to one of Claims 1 to 6, **characterized in that** said diamide bears an amide function based on a hydroxy acid according to b) and another function based on said monoacid c) corresponding to a mole ratio b)/c) of 1/1.

8. Amide according to one of Claims 1 to 7, **characterized in that** it comprises at least two, preferably at least three different reaction products, as derived from the reaction in said reaction mixture of an amine a) or d) with a monoacid b) or c).

9. Amide according to Claim 8, **characterized in that** it is a diamide and **in that** it comprises a mixture of products having the following formulae:
- b1-a1-c1
- b1-a1-b1
and preferably:
- b1-a1-c1
- b1-a1-b1
- b1-a2-b1
with a1: diamine residue according to a) bearing two amide groups -NHC(=O)-, a2: diamine residue according to d) bearing two groups -NHC(=O)-, b1: residue of the acid 14-HEA without a carboxyl group and c1: non-hydroxylated monoacid residue according to c) without a carboxyl group.

10. Use of the amide, as defined according to one of Claims 1 to 9, **characterized in that** it is a use as an organogelling agent.

11. Use according to Claim 10, **characterized in that** said amide is used in coating, bonding or adhesive, molding, mastic or sealing agent compositions or cosmetic compositions.

12. Organogelling agent, **characterized in that** it comprises at least one diamide as defined according to one of Claims 1 to 9.

13. Organogelling agent according to Claim 12, **characterized in that** it is a thixotropic rheology agent or additive in a composition preconcentrated in an organic plasticizer or in an organic solvent, in preactivated paste form.

14. Organic binder composition, **characterized in that** it comprises as rheology agent at least one amide as defined according to one of Claims 1 to 9.

15. Organic binder composition according to Claim 14, **characterized in that** said organic binder is a binder for coating compositions from paints, varnishes, inks and gelled renderings or a binder for bonding or adhesive compositions or a binder for mastic or sealing agent or stripping agent compositions or for molding compositions or cosmetic compositions.

16. Composition according to Claim 14 or 15, **characterized in that** said binder is selected from: epoxy resins, unsaturated and saturated polyesters, vinyl esters, alkyds, silane-based resins, polyurethanes, polyesteramides, solvent-based acrylic resins, multifunctional acrylic monomers and/or oligomers or acryl acrylic resins with reactive diluent or inert resins diluted in a reactive or non-reactive solvent.
